# EUROPEAN PATENT SPECIFICATION

(11) **EP 0 527 628 B1**
(45) Date of publication and mention of the grant of the patent: **03.07.1996**
(21) Application number: 92307307.6
(22) Date of filing: 10.08.1992
(51) Int. Cl.: C12Q 1/68, C07H 21/00, C12Q 1/14

(54) **Rapid method for detection of methicillin-resistant staphylococci**
Schnelle Methode zum Nachweis von methicillin-resistenten Staphylokokken
Méthode rapide de détection de staphylocoques résistants à la méthycilline

(30) Priority: 13.08.1991 US 744770
(43) Date of publication of application: 17.02.1993
(73) Proprietor: ELI LILLY AND COMPANY, Indianapolis, Indiana 46285 (US)
(72) Inventor: Skatrud, Paul Luther, Greenwood, Indiana (US); Unal, Serhat, Ankara (TR)
(74) Representative: Hudson, Christopher Mark

(56) References cited:
- WO-A-91/08305
- WO-A-92/05281
- CHEMICAL ABSTRACTS, vol. 102, no. 15, 15 April 1985, Columbus, OH (US); W.F. NAUSCHUETZ et al., p. 335, no. 128675r; and A.P. BARTON, pp. 335-336, no. 128684t
- CHEMICAL ABSTRACTS, vol. 101, no. 1, 02 July 1984, Columbus, OH (US); D.J. FLOURNOY et al., p.329, no. 3832n/

## Description

Methicillin resistance in staphylococci is an important unversal problem in hospitals and geriatric care centers. Both methicillin-resistant Staphylococcus aureus (MRSA) and methicillin-resistant Staphylococcus epidermis (MRSE) are common nosocomial pathogens. The infections they cause are serious and difficult to treat. Horan et al., Morbid Mortal. Weekly Rep. 35:1755 (1984); Maple et al., Lancet i:537 (1989). Only a few antibiotics are available for treatment and they all have undesirable side effects. Hackbarth and Chambers, Antimicrob. Agents Chemother. 33:995 (1989). Currently, time-consuming, labor intensive, and somewhat unreliable methods are employed for the detection of MRSA/MRSE. Id These methods include disk diffusion, broth dilution and agar screening. Id Such methods do not reliably detect heterogeneously resistant staphylococci. Thus, it is imperative to develop a rapid, standardized, accurate and sensitive method for the detection of methicillin resistance in staphylococci.

MRSA/MRSE carry the mecA gene which encodes penicillin binding protein 2a (PBP2a). This proteins responsible for the phenotypic expression of methicillin resistance in staphylococci. Chambers, Antimicrob. Agents Chemother. 33:424 (1989); Hackbarth and Chambers, Antimicrob. Agents Chemother. 33:991 (1989); Tonin and Tomasz, Antimicrob. Agents. Chemother. 30:577 (1986). Staphylococcal strains susceptible to methicillin do not harbor a mecA gene. Therefore, the mecA, gene is a useful molecular handle for rapid identification of MRSA/MRSE. Detection of mecA by DNA hybridization has provided a relatively sensitive method for identifying MRSA/MRSE strains. Archer and Penell, Antimicrob. Agents Chemother. 34:1720 (1990); Lencastre et al,. Antimicrob. Agents Chemother. 35:575 (1991). However, DNA hybridization suffers from several disadvantages. A large number of cells are required, DNA extraction and immobilization on a membrane is a time-consuming process, and frequently radioactive isotopes are employed, although recently non-radioactive probes have become available. Ligozzi et al., Antimicrob. Agents Chemother. 35:575 (1991).

The present invention provides a method for detecting methicillin-resistant staphylococcal infections, said method comprising:
a) performing the polymerase chain reaction on clinical samples suspected of staphylococcal infection, said polymerase chain reaction being primed by DNA primers composed of two oligonucleotides of high G+C content, wherein one oligonucleotide has a DNA sequence comprised by the coding strand of a Staphylococcus mecA gene and the second DNA primer has a DNA sequence comprised by the non-coding strand of a Staphylococcus mecA gene; and
b) analyzing the reaction product of step a.

The present invention also provides a method for the rapid release of DNA from staphylococci, said method comprising:
a) treating a sample containing staphylococci with lysostaphin;
b) treating the resultant sample of step a with proteinase K; and
c) incubating the resultant sample of step b in a boiling water bath.

Additionally, the present invention provides a method for detecting methicillin-resistant staphylococcal infections in a sample of interest, said method comprising:
a) treating a sample of interest with lysostaphin;
b) treating the resultant sample of step a with proteinase K;
c) incubating the resultant sample of step b in a boiling water bath;
d) performing the polymerase chain reaction on the resultant sample of step c, said polymerase chain reaction being primed by DNA primers, said DNA primers being composed of two oligonucleotides of high G+C content, wherein one oligonucleotide has a DNA sequence comprised by the coding strand of a Staphylococcus mecA gene and the second DNA primer has a DNA sequence comprised by the non-coding strand of a Staphylococcus mecA gene; and
e) analyzing the reaction product of step d.

For purposes of the present invention the following terms are defined below:
High G+C content - G+C content significantly higher than the average 30.5% G+C content of staphylococcal mecA genes.
mecA gene - a gene encoding penicillin binding protein 2A, which is responsible for methicillin resistance.
Methicillin-resistant staphylococci - staphylococci which are resistant to all beta-lactams, including cephalosporins and penicillin derivatives such as methicillin and oxacillin.
PBP2A - penicillin binding protein 2A.

The present invention provides a method for detecting methicillin-resistant staphylococcal infections, said method comprising:
a) performing the polymerase chain reaction on clinical samples suspected of staphylococcal infection, said polymerase chain reaction being primed by DNA primers, said DNA primers being composed of two oligonucleotides of high G+C content, wherein one oligonucleotide has a DNA sequence comprised by the coding strand of a Staphylococcus mecA gene and the second DNA primer has a DNA sequence comprised by the non-coding strand of a Staphylococcus mecA gene; and
b) analyzing the reaction product of step a.

The polymerase chain reaction (PCR) is now well-known in the art as a route to the amplification of minute quantities of DNA. See U.S. Patents 4,683,195, 4,800,159 and 4, 683,202. The technique has been applied in clinical settings to the detection of a variety of different pathogens. See. e.g., Cassol et al., J. Clin. Microbiol. 29:667-671 (1991); Lopez et al., J. Clin. Microbiol. 29:578-582 (1991); Brisson-Noel et al., Lancet ii:1069-1071 (1989); Valentine et al., J. Clin. Microbiol. 29:689-695 (1991); and Gouvea et al., J. Clin. Microbiol. 29:529-523 (1991). The technique has been used to detect toxins of Staphylococcus aureus but until the present invention the technique was not applied to the detection methicillin-resistant staphylococci.

Staphylococci resistant to beta-lactams, particularly Staphylococcus aureus and Staphylococcus epidermidis, represent a significant and steadily increasing problem for physicians. Due to the speed with which these bacteria can cause death, it is advantageous to quickly ascertain whether the organisms present in a patient are non-responsive to betalactams. The present invention provides a method which can be performed significantly faster than prior art methods (Hackbarth and Chambers, Antimicrob. Agents and Chemother. 33:995-999 (1989)), including methods based on DNA hybridization (Ligozzi et al., Antimicrob. Agents and Chemother. 35:575-578 (1991) and Archer and Pennel, Antimicrob. Agents and Chemother. 34:1720-1724 (1990)). The extreme sensitivity afforded by the PCR is another vital aspect of the present invention. The methicillin-resistant staphylococci are open heterogeneously resistant. Thus, non-DNA based resistance determinations can give inconsistent results.

Penicillin binding protein 2A confers methicillin resistance by having a low affinity for the beta-lactams. The proteins encoded by the mecA gene. The present invention will enable the detection of any staphylococci bearing the mecA gene. The clinically relevant staphylococcal strains are primarily S. epidermidis and S. aureus and, occasionally, S. haemolyticus. Rarely problematic strains are S. simulans, S. carnosus and S. saprophyticus. All the mecA genes which have been isolated and sequenced have a very high similarity (>99%), thus allowing the use of one or two sets of primers to detect all mecA-containing strains. If primers are made from regions of the gene which are known to contain variance among mecA genes isolated from different sources, the length of the primers used should be at least thirty nucleotides to ensure specific priming. These regions correlate to nucleotides 605-607, 615-617, 697-699, 746-747, 841, 1010-1011, 1819, and 1933 of SEQ ID NO:1.

The average G+C content of the known mecA genes is about 30.5%. To ensure specific priming, the G+C content of the DNA primers used for the PCR should be significantly higher than 30.5%, preferably about 50% or higher. The sequences of the DNA primers may be derived from any mecA gene. The sequences of some of these genes can be found in Song et al., FEBS Letters 221:167-171 (1987) (S. aureus TK784) and Ryffel et al., Gene 94:137-38 (1990) (S. aureus BB270 and S. epidermidis WT55). Additionally, the DNA primers may be derived from the S. aureus 27R mecA gene, set out as SEQ ID NO:1.

Skilled artisans will recognize that, within a particular set of primers, one of the oligonucleotides should be derived from the coding strand of the gene and the other oligonucleotide should be derived from the non-coding strand. Preferred DNA primers have sequences corresponding to nucleotides 141-160 of SEQ ID NO:1 and the inverse complement of nucleotides 1929-1952 of SEQ ID NO:1. The primers can be synthesized by the modified phosphotriester method using fully protected deoxyribonucleotide building blocks. Such synthetic methods are well known in the art and can be carried out in substantial accordance with Itakura et al., Science 198:1056 (1977), Crea et al., Proc. Nat'l Acad. Sci. USA 75:5765 (1978), Hsiung et al., Nuc. Acids Res. 11:3227 (1983) or Narang et al., Methods in Enzymology 68:90 (1980). An especially preferred method employs automated DNA synthesizers such as the Applied Biosystems 380B DNA synthesizer (850 Lincoln Centre Drive, Foster City, CA 94404).

Protocols for performing the PCR reaction are set out in PCR Protocols: A Guide to Methods and Applications, ed. Michael A. Innis et al., Academic Press, Inc., 1990. An especially preferred protocol is described in the Examples herein. The results of the reaction may be analyzed in any way desired; a preferred method is by agarose gel electrophoresis. It may be desirable to further confirm the presence of staphylococci comprising the mecA gene by performing "nested PCR." In this technique, two consecutive PCRs are performed. The oligonucleotides used to prime the second PCR should be derived from DNA sequences of the mecA gene interior to the DNA sequences on which the first set of primers are based. Preferred DNA primers for the second PCR have sequences corresponding to nucleotides 568-593 of SEQ ID NO:1 and the inverse complement of nucleotides 1647-1670 of SEQ ID NO:1.

The present invention also provides a method for the rapid release of DNA from staphylococci, said method comprising:
a) treating a sample containing staphylococci with lysostaphin;
b) treating the resultant sample of step a with proteinase K; and
c) incubating the resultant sample of step b in a boiling water bath. The method of the present invention is much faster than prior art staphylococcal DNA extraction methods. A preferred embodiment of the rapid release method is outlined in the Examples. The method is particularly useful in combination with the methicillin-resistant staphylococci detection technique of the present invention. Thus, the present invention also provides a method for detecting methicillin-resistant staphylococcal infections in a sample of interest, said method comprising:
   a) treating a sample interest with lysostaphin;
   b) treating the resultant sample of step a with proteinase K;
   c) incubating the resultant sample of step b in a boiling water bath;
   d) performing the polymerase chain reaction on the resultant sample of step c) said polymerase chain reaction being primed by DNA primers, said DNA primers being composed of two oligonucleotides of high G+C content, wherein one oligonucleotide has a DNA sequence comprised by the coding strand of a Staphylococcus mecA gene and the second DNA primer has a DNA sequence comprised by the non-coding strand of a Staphylococcus mecA gene; and
   e) analyzing the reaction product of step d.

This method represents a combination of the rapid release DNA extraction method and the method for detecting methicillin-resistant staphylococci outlined above. The various considerations discussed regarding those two techniques are equally applicable when the methods are used in combination. The detection of methicillin-resistant staphylococci via the present invention requires only three hours. In contrast, when the prior art DNA extraction method is used in lieu of the rapid release DNA extraction method, six hours are required. Any additional time is significant in potential life or death situations such as methicillin-resistant staphylococcal infections. The speed and sensitivity of the present invention allows one to avoid empirical antibiotic therapy, which is fraught with liabilities such as high cost, toxicity factors and resistance development.

The present invention may be used to detect methicillin-resistant staphylococci in any internal body fluid, including blood, urine, spinal fluid and fluid drained from an abscess. If desired, the samples may be cultured and the PCR performed on a sample of the bacteria from the growth medium. The use of the PCR on body fluids is known in the prior art. See, e.g., Cassol et al., J. Clin. Microbiol. 29:667-671 (1991); Lopez et al., J. Clin. Microbiol. 29:578-582 (1991); and Brisson-Noel et al., Lancet ii:1069-1071 (1989).

The following Examples are intended to further illustrate and exemplify, but not limit the scope of, the present invention.

### Example 1

A total of 70 staphylococcal strains (33 S. aureus and 37 S. epidermidis) isolated from various clinical settings were examined. Species identification was done with a Staph-Ident™ Diagnostic Kit (Analytab, Sherwood Medical, Plainview, NY). To verify the results obtained with the PCR, all were screened for methicillin resistance by growth on Mueller-Hinton agar (Difco, Detroit, Ml) supplemented with 4% sodium chloride and 6 µg oxacillin/ml. Inoculated plates were incubated for 24 hours at 35 °C and examined for the presence of growth. Sixteen of 33 S. aureus and 27 of 37 S. epidermidis were categorized as methicillin-resistant using this method.

### Example 2

Each strain examined for methicillin resistance in Example 1, above, was treated as follows. Bacteria were harvested from either TY agar plates (one loopful) or TY broth cultures (one ml of overnight culture diluted to 10⁸ bacteria/ml). Cells from broth medium were harvested by centrifugation for 30 seconds in a microcentrifuge. Cells from either source were resuspended in 50 µl of lysostaphin solution (100 µg/ml in water, Sigma Chemical Co., St. Louis, MO). Cell suspensions were incubated at 37 °C. After 10 minutes, 50 µl proteinase K solution (100 µg/ml in water, Sigma), and 150 µl of 0.1 M Tris, pH 7.5, were added. The cell suspensions were incubated at 37 °C for an additional 20 minutes and then placed in a boiling water bath for 10 minutes. This treatment effectively lysed S. aureus or S. epidermidis cells and prevented DNase activity. Ten µl from these cell lysates were used directly in PCRs.

### Example 3

Two primers were chosen for the PCR that were separated by 1.8 kb in the mecA open reading frame. The sequences of the two primers were 5′-GTTGTAGTTGTCGGGTTTGG-3′(nucleotides 141-160 of SEQ ID NO:1) and 5′-CCACCCAATTTGTCTGCCAGTTTCTCC-3′(inverse complement of nucleotides 1929-1952 of SEQ ID NO:1). The PCR was performed in a DNA Thermal Cycler using a Gene Amp Kit according to the manufacturer's instructions (Perkin Elmer Cetus, Norwalk, CT). A thermal step program that included the following parameters was used for DNA amplification: denaturation at 94 °C for 30 seconds, annealing at 55 °C for 30 seconds, primer extension at 72 °C for two minutes, for a total of 30 cycles. Ten µl of the PCR solution were analyzed by electrophoresis on a 0.8% agarose gel. A positive result was indicated by the presence of a 1.8 kb amplified DNA fragment. In all, 69 of the 70 staphylococcal strains produced exactly the same result with PCR and the oxacillin susceptibility test. Examination of the single exception revealed that it was a mixed culture containng a rapidly growing methicillin-sensitive strain (mecA⁻⁾ and a slower growing methicillin-resistant (mecA⁺) strain. When the strains were separated the susceptibility test and the PCR were in agreement. Subsequent nested PCR analysis of the intial DNA sample with the internal DNA primers (corresponding to nucleotides 568-593 of SEQ ID NO:1 and the inverse complement of nucleotides 1647-1670 of SEQ ID NO:1) produced a positive PCR result. No isolates were found to be mecA sensitive but methicillin resistant. These results emphasize the fact that a positive result in a DNA-based test correlates very well with the methicillin-resistant phenotype.

## Claims

1. A method for detecting methicillin resistant staphylococcal infections, said method comprising:
a) performing the polymerase chain reaction on clinical samples suspected of staphylococcal infection, said polymerase chain reaction being primed by DNA primers, said DNA primers being composed of two oligonucleotides of high GC content, wherein one oligonucleotide has a DNA sequence comprised by the coding strand of a Staphylococcus mecA gene and the second DNA primer has a DNA sequence comprised by the non-coding strand of a Staphylococcus mecA gene; and
b) analyzing the reaction product of step a.

2. The method of Claim 1 wherein the GC content of the DNA primers is approximately 50% or greater.

3. The method of Claim 2 wherein the sequences of the DNA primers are nucleotides 141-160 of SEQ ID NO:1 and the inverse complement of nucleotides 1929-1952 of SEQ ID NO:1.

4. The method of Claim 1 wherein an additional subsequent polymerase chain reaction is performed using DNA primers interior to the DNA primers used in the initial polymerase chain reaction.

5. The method of Claim 4 wherein the sequences of the interior DNA primers are nucleotides 568-593 of SEQ ID NO:1 and the inverse complement of nucleotides 1647-1670 of SEQ ID NO:1.

6. The method of Claim 1 wherein the sequence of the DNA primers is comprised by the coding and non-coding strands of a gene selected from a group consisting of Staphylococcus aureus and Staphylococcus epidermidis mecA genes.

7. The method of Claim 6 wherein the gene is selected from a group consisting of the S. aureus 27R, S. aureus BB270, S. aureus TK784, and the S. epidermidis WT55 mecA genes.

8. The method of Claim 1 wherein the method is used to detect methicillin resistant staphylococci selected from the group consisting of S. aureus, S. epidermidis, S. haemolyticus, S. simulans, S. carnosus, and S. saprophyticus.

9. The method of Claim 1 wherein the reaction product of step a) is analyzed by gel electrophoresis.

10. A method for the rapid release of DNA from staphylococci, said method comprising:
a) treating a sample containing staphylococci with lysostaphin;
b) treating the resultant sample of step a with proteinase K; and
c) incubating the resultant sample of the step in a boiling water bath.

11. The method of Claim 10 wherein the sample containing staphylococci is derived from blood, urine, spinal fluid, an abscess or bacteriological growth medium.

12. A method for detecting methicillin resistant staphylococcal infections in a sample of interest, said method comprising:
a) treating a sample of interest with lysostaphin;
b) treating the resultant sample of step a with proteinase K;
c) incubating the resultant sample of step b in a boiling water bath;
d) performing the polymerase chain reaction on the resultant sample of step c) said polymerase chain reaction being primed by DNA primers, said DNA primers being composed of two oligonucleotides of high GC content, wherein one oligonucleotide has a DNA sequence comprised by the coding strand of a Staphylococcus mecA gene and the second DNA primer has a DNA sequence comprised by the non-coding strand of a Staphylococcus mecA gene; and
e) analyzing the reaction product of step d.

13. The method of Claim 12 wherein the GC content of the DNA primers is approximately 50% or greater.

14. The method of Claim 13 wherein the sequences of the DNA primers are nucleotides 141-160 of SEQ ID NO:1 and the inverse complement of nucleotides 1929-1952 of SEQ ID NO:1.

15. The method of Claim 14 wherein an additional subsequent polymerase chain reaction is performed using DNA primers interior to the DNA primers used in the initial polymerase chain reaction.

16. The method of Claim 15 wherein the sequences of the interior DNA primers are nucleotides 568-593 of SEQ ID NO:1 and the inverse complement of nucleotides 1647-1670 of SEQ ID NO:1

17. The method of Claim 12 wherein the sequence of the DNA primers is comprised by the coding and non-coding strands of a gene selected from a group consisting of Staphylococcus aureus and Staphylococcus epidermidis mecA genes.

18. The method of Claim 17 wherein the sequence of the DNA primers is comprised by the coding and non-coding stands of a gene selected from a group consisting of the S. aureus 27R, S. aureus BB270, S. aureus TK784, and the S. epidermis WT55 mecA genes.

19. The method of Claim 12 wherein the method is used to detect methicilin-resistant staphylococci selected from the group consisting of S. aureus, S. epidermidis, S. haemolyticus, S. simulans, S. carnosus, and S. saprophyticus.

20. The method of Claim 12 wherein the reaction product of step d) is analyzed by gel electrophoresis.

21. The method of Claim 12 wherein the sample of interest is derived from blood, urine, spinal fluid, an abscess or bacteriological growth medium.

## Patentansprüche

1. Verfahren zur Detektion von Methicillin-resistenten Staphylokokkeninfektionen, gekennzeichnet durch:
a) Durchführung der Polymerasekettenreaktion mit klinischen Proben, die unter dem Verdacht der Staphylokokkeninfektion stehen, wobei die Polymerasekettenreaktion durch DNA Primer geprimt wird, wobei diese DNA Primer sich aus zwei Oligonukleotiden mit hohem G+C Gehalt zusammensetzen, worin ein Oligonukleotid eine DNA Sequenz aufweist, die auf dem kodierenden Strang eines Staphylococcus mecA Gens enthalten ist und der zweite DNA Primer eine DNA Sequenz aufweist, die auf dem nicht-kodierenden Strang eines Staphylococcus mecA Gens enthalten ist, und
b) Analyse des Reaktionsprodukts von Schritt a.

2. Verfahren nach Anspruch 1, worin der G+C-Gehalt der DNA Primer etwa 50 % oder mehr beträgt.

3. Verfahren nach Anspruch 2, worin die Sequenzen der DNA Primer die Nukleotide 141-160 von SEQ ID Nr.1 und das inverse Komplementär der Nukleotide 1929-1952 von SEQ ID Nr. 1 sind.

4. Verfahren nach Anspruch 1, worin zusätzlich eine weitere Polymerasekettenreaktion mittels DNA Primern durchgeführt wird, die innerhalb der DNA Primer liegen, welche bei der anfänglichen Polymerasekettenreaktion verwendet wurden.

5. Verfahren nach Anspruch 4, worin die innerhalb liegenden DNA Primer die Nukleotide 568-593 von SEQ ID Nr. l und das inverse Komplementär der Nukleotide 1647-1670 von SEQ ID Nr. 1 sind.

6. Verfahren nach Anspruch 1, worin die Sequenz der DNA Primer sich aus den kodierenden und den nicht-kodierenden Strängen eines Gens zusammensetzen, das aus der Gruppe ausgewählt ist, die aus Staphylococcus aureus und Staphylococcus epidermidis mecA Genen besteht.

7. Verfahren nach Anspruch 6, worin das Gen aus einer Gruppe ausgewählt ist, die aus S. aureus 27R, S. aureus BB270, S. aureus TK784 und S. idermidis WT55 mecA Genen besteht.

8. Verfahren nach Anspruch 1, worin das Verfahren verwendet wird, um die Methicillin-resistenten Staphylokokken zu detektieren, die aus der Gruppe ausgewählt sind, welche besteht aus S. aureus, S. epidermidis, S. haemolyticus, S. simulans, S. carnosus und S. saprophyticus.

9. Verfahren nach Anspruch 1, worin das Reaktionsprodukt von Schritt a) durch Gelelektrophorese analysiert wird.

10. Verfähren zur schnellen Freisetzung von DNA aus Staphylokokken, gekennzeichnet durch:
a) Behandlung einer Staphylokokken enthaltenden Probe mit Lysostaphin,
b) Behandlung der entstehenden Probe von Schritt a) mit Proteinase K und
c) Inkubation der entstehenden Probe von Schritt b) in einem kochenden Wasserbad.

11. Verfahren nach Anspruch 10, worin die Staphylokokken enthaltende Probe aus Blut, Urin, Spinalflüssigkeit, einem Abszess oder einem bakteriologischen Wachstumsmedium stammt.

12. Verfahren zur Detektion von Methicillin-resistenten Staphylokkenüfiektionen in einer Probe von Interesse, gekennzeichnet durch:
a) Behandlung der Probe von Interesse mit Lysostaphin,
b) Behandlung der entstehenden Probe von Schritt a) mit Proteinase K
c) Inkubation der entstehenden Probe von Schritt b) in einem kochenden Wasserbad.
d) Durchführung der Polymerasekettenreaktion mit der entstehenden Probe von Schritt c), wobei die Polymerasekettenreaktion durch DNA Primer geprimt wird, wobei diese DNA Primer sich aus zwei Oligonukleotiden mit hohem G+C Gehalt zusammensetzen, worin ein Oligonukleotid eine DNA Sequenz aufweist, die auf dem kodierenden Strang eines Staphylococcus mecA Gens enthalten ist und der zweite DNA Primer eine DNA Sequenz aufweist, die auf dem nicht-kodierenden Strang eines Staphylococcus mecA Gens enthalten ist, und
e) Analyse des Reaktionsprodukts von Schritt d.

13. Verfahren nach Anspruch 12, worin der G+C-Gehalt der DNA Primer etwa 50 % oder mehr beträgt.

14. Verfahren nach Anspruch 13, worin die Sequenzen der DNA Primer die Nukleotide 141-160 von SEQ ID Nr.1 und das inverse Komplementär der Nukleotide 1929-1952 von SEQ ID Nr.2 sind.

15. Verfahren nach Anspruch 14, worin zusätzlich eine weitere Polymerasekettenreaktion mittels DNA Primern durchgeführt wird, die innerhalb der DNA Primer liegen, welche bei der anfänglichen Polymerasekettenreaktion verwendet wurden.

16. Verfahren nach Anspruch 15, worin die innerhalb liegenden DNA Primer die Nukleotide 568-593 von SEQ ID Nr. 1 und das inverse Komplementär der Nukleotide 1647-1670 von SEQ ID Nr. 1 sind.

17. Verfahren nach Anspruch 12, worin die Sequenz der DNA Primer sich aus den kodierenden und den nicht-kodierenden Strängen eines Gens zusammensetzen, das aus der Gruppe ausgewählt ist, die aus Staphylococcus aureus und Staphylococcus epidermidis mecA Genen besteht.

18. Verfahren nach Anspruch 17, worin die DNA Sequenz der DNA Primer auf den kodierenden und nicht-kodierenden Strängen eines Gens enthalten sind, das aus einer Gruppe ausgewählt ist, die aus S. aureus 27R, S. aureus BB270, S. aureus TK784 und S. epidermidis WT55 mecA Genen besteht.

19. Verfahren nach Anspruch 12, worin das Verfahren verwendet wird, um die Methicillin-resistenten Staphylokokken zu detektieren, die aus der Gruppe ausgewählt sind, welche besteht aus S. aureus, S. epidermidis, S. haemolyticus, S. simulans, S. carnosus und S. saprophyticus

20. Verfahren nach Anspruch 12, worin das Reaktionsprodukt von Schritt d) durch Gelelektrophorese analysiert wird.

21. Verfahren nach Anspruch 12, worin die Probe von Interesse aus Blut, Urin, Spinalflüssigkeit, einem Abszess oder einem bakteriologischen Wachstumsmedium stammt.

## Revendications

1. Méthode pour la détection d'infections par des staphylocoques résistants à la méthycilline, ladite méthode comprenant :
a) la réalisation de la réaction en chaîne par la polymérase sur des échantillons cliniques soupçonnés d'être infectés par des staphylocoques, ladite réaction en chaîne par la polymérase étant amorcée par des amorces d'ADN, lesdites amorces d'ADN étant composées de deux oligonucléotides à teneur élevée en GC, dans lesquelles un oligonucléotide présente une séquence d'ADN comprise dans le brin codant d'un gène mecA de Staphylococcus et la deuxième amorce d'ADN présente une séquence d'ADN comprise dans le brin non-codant d'un gène mecA de Staphylococcus; et
b) l'analyse du produit de réaction de l'étape a.

2. Méthode selon la revendication 1, dans laquelle la teneur en GC des amorces d'ADN vaut approximativement 50 % ou davantage.

3. Méthode selon la revendication 2, dans laquelle les séquences des amorces d'ADN sont les nucléotides 141 - 160 de SEQ ID NO:1 et le complément inverse des nucléotides 1929 - 1952 de SEQ ID NO:1.

4. Méthode selon la revendication 1, dans laquelle une réaction en chaîne par polymérase subséquente supplémentaire est effectuée en utilisant des amorces d'ADN intérieures aux amorces d'ADN utilisées dans la réaction en chaîne par polymérase initiale.

5. Méthode selon la revendication 4, dans laquelle les séquences des amorces d'ADN intérieures sont les nucléotides 568 - 593 de SEQ ID NO:1 et le complément inverse des nucléotides 1647 - 1670 de SEQ ID NO:1.

6. Méthode selon la revendication 1, dans laquelle la séquence des amorces d'ADN est comprise dans les brins codant et non-codant d'un gène choisi parmi un groupe constitué des gènes mecA de Staphylococcus aureus et de Staphylococcus epidermidis.

7. Méthode selon la revendication 6, dans laquelle le gène est choisi parmi un groupe constitué des gènes mecA de S. aureus 27R, de S. aureus BB270,de S. aureus TK784, et de S. epidermidis WT55.

8. Méthode selon la revendication 1, dans laquelle la méthode est utilisée pour détecter des staphylocoques résistants à la méthycilline choisis parmi le groupe constitué de S. aureus, de S. epidermidis, de S. haemolyticus, de S. simulans, de S. carnosus et de S. saprohyticus.

9. Méthode selon la revendication 1, dans laquelle le produit de réaction de l'étape a) est analysé par électrophorèse sur gel.

10. Méthode pour la libération rapide d'ADN à partir de staphylocoques, ladite méthode comprenant :
a) le traitement d'un échantillon contenant des staphylocoques avec la lysostaphine;
b) le traitement de l'échantillon résultant de l'étape a par la protéinase K; et
c) l'incubation de l'échantillon résultant de l'étape b dans un bain d'eau bouillante.

11. Méthode selon la revendication 10, dans laquelle l'échantillon contenant des staphylocoques est dérivé du sang, de l'urine, du liquide spinal, d'un abcès ou d'un milieu de croissance bactériologique.

12. Méthode pour la détection d'infections par des staphylocoques résistants à la méthycilline dans un échantillon d'intérêt, ladite méthode comprenant :
a) le traitement d'un échantillon d'intérêt par la lysostaphine;
b) le traitement de l'échantillon résultant de l'étape a par la protéinase K;
c) l'incubation de l'échantillon résultant de l'étape b dans un bain d'eau bouillante;
d) la réalisation de la réaction en chaîne par la polymérase sur l'échantillon résultant de l'étape c, ladite réaction en chaîne par la polymérase étant amorcée par des amorces d'ADN, lesdites amorces d'ADN étant composées de deux oligonucléotides à teneur élevée en GC, dans lesquelles un oligonucléotide présente une séquence d'ADN comprise dans le brin codant d'un gène mecA de Staphylococcus et la deuxième amorce d'ADN présente une séquence d'ADN comprise dans le brin non-codant d'un gène mecA de Staphylococcus; et
e) l'analyse du produit de réaction de l'étape d.

13. Méthode selon la revendication 12, dans laquelle la teneur en GC des amorces d'ADN vaut approximativement 50 % ou davantage.

14. Méthode selon la revendication 13, dans laquelle les séquences des amorces d'ADN sont les nucléotides 141 - 160 de SEQ ID NO:1 et le complément inverse des nucléotides 1929 - 1952 de SEQ ID NO:1.

15. Méthode selon la revendication 14, dans laquelle une réaction en chaîne par polymérase subséquente supplémentaire est effectuée en utilisant des amorces d'ADN intérieures aux amorces d'ADN utilisées dans la réaction en chaîne par polymérase initiale.

16. Méthode selon la revendication 15, dans laquelle les séquences des amorces d'ADN intérieures sont les nucléotides 568 - 593 de SEQ ID NO:1 et le complément inverse des nucléotides 1647 - 1670 de SEQ ID NO:1.

17. Méthode selon la revendication 12, dans laquelle la séquence des amorces d'ADN est comprise dans les brins codant et non-codant d'un gène choisi parmi un groupe constitué des gènes mecA de Staphylococcus aureus et de Staphylococcus epidermidis.

18. Méthode selon la revendication 17, dans laquelle la séquence des amorces d'ADN est comprise dans les brins codant et non-codant d'un gène choisi parmi un groupe constitué des gènes mecA de S. aureus 27R, S. aureus BB270, S. aureus TK784, et S. epidermidis WT55.

19. Méthode selon la revendication 12, dans laquelle la méthode est utilisée pour détecter des staphylocoques résistants à la méthycilline choisis parmi le groupe constitué de S. aureus, de S. epidermidis, de S. haemolyticus, de S. simulans, de S. carnosus et de S. saprohyticus.

20. Méthode selon la revendication 12, dans laquelle le produit de réaction de l'étape d) est analysé par électrophorèse sur gel.

21. Méthode selon la revendication 12, dans laquelle l'échantillon d'intérêt est dérivé du sang, de l'urine, du liquide spinal, d'un abcès ou d'un milieu de croissance bactériologique.


### SEQUENCE LISTING

(1) GENERAL INFORMATION:
   (i) APPLICANT: Eli Lilly and Company
   (ii) TITLE OF INVENTION: Rapid Method for Detection of Methicillin Resistant Staphylococci
   (iii) NUMBER OF SEQUENCES: 2
   (iv) CORRESPONDENCE ADDRESS:
      (A) ADDRESSEE: C. M. Hudson
      (B) STREET: Erl Wood Manor
      (C) CITY: Windlesham
      (D) STATE: Surrey
      (E) COUNTRY: United Kingdom
      (F) ZIP: GU20 6PH
   (v) ATTORNEY/AGENT INFORMATION:
      (A) NAME: C. M. Hudson
      (B) REGISTRATION NUMBER: 307
      (C) REFERENCE/DOCKET NUMBER: X-8573
   (vi) TELECOMMUNICATION INFORMATION:
      (A) TELEPHONE: 0276 78441
      (B) TELEFAX: 0276 78306
(2) INFORMATION FOR SEQ ID NO:1:
   (i) SEQUENCE CHARACTERISTICS:
      (A) LENGTH: 2111 base pairs
      (B) TYPE: nucleic acid
      (C) STRANDEDNESS: both
      (D) TOPOLOGY: linear
   (ii) MOLECULE TYPE: DNA (genomic)
   (iii) HYPOTHETICAL : NO
   (iv) ANTI-SENSE: NO
   (ix) FEATURE:
      (A) NAME/KEY: CDS
      (B) LOCATION: 105..2111
   (xi) SEQUENCE DESCRIPTION: SEQ ID NO:1:
(2) INFORMATION FOR SEQ ID NO:2:
   (i) SEQUENCE CHARACTERISTICS:
      (A) LENGTH: 668 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Lys Lys Ile Lys Ile Val Pro Leu Ile Leu Ile Val Val Val Val
    1               5                  10                  15
   Gly Phe Gly Ile Tyr Phe Tyr Ala Ser Lys Asp Lys Glu Ile Asn Asn
               20                  25                  30
   Thr Ile Asp Ala Ile Glu Asp Lys Asn Phe Lys Gln Val Tyr Lys Asp
           35                  40                  45
   Ser Ser Tyr Ile Ser Lys Ser Asp Asn Gly Glu Val Glu Met Thr Glu
       50                  55                  60
   Arg Pro Ile Lys Ile Tyr Asn Ser Leu Gly Val Lys Asp Ile Asn Ile
   65                  70                  75                  80
   Gln Asp Arg Lys Ile Lys Lys Val Ser Lys Asn Lys Lys Arg Val Asp
                   85                  90                  95
   Ala Gln Tyr Lys Ile Lys Thr Asn Tyr Gly Asn Ile Asp Arg Asn Val
               100                 105                 110
   Gln Phe Asn Phe Val Lys Glu Asp Gly Met Trp Lys Leu Asp Trp Asp
           115                 120                 125
   His Ser Val Ile Ile Pro Gly Met Gln Lys Asp Gln Ser Ile His Ile
       130                 135                 140
   Glu Asn Leu Lys Ser Glu Arg Gly Lys Ile Leu Asp Arg Asn Asn Val
   145                 150                 155                 160
   Glu Leu Ala Asn Thr Gly Thr Ala Tyr Glu Ile Gly Ile Val Pro Lys
   165 170 175
   Asn Val Ser Lys Lys Asp Tyr Lys Ala Ile Ala Lys Glu Leu Ser Ile
   180 185 190
   Ser Glu Asp Tyr Ile Lys Gln Gln Met Asp Gln Asn Trp Val Gln Asp
   195 200 205
   Asp Thr Phe Val Pro Leu Lys Thr Val Lys Lys Met Asp Glu Tyr Leu
   210 215 220
   Ser Asp Phe Ala Lys Lys Phe His Leu Thr Thr Asn Glu Thr Glu Ser
   225 230 235 240
   Arg Asn Tyr Pro Leu Glu Lys Ala Thr Ser His Leu Leu Gly Tyr Val
   245 250 255
   Gly Pro Ile Asn Ser Glu Glu Leu Lys Gln Lys Glu Tyr Lys Gly Tyr
   260 265 270
   Lys Asp Asp Ala Val Ile Gly Lys Lys Gly Leu Glu Lys Leu Tyr Asp
   275 280 285
   Lys Lys Leu Gln His Glu Asp Gly Tyr Arg Val Thr Ile Val Asp Asp
   290 295 300
   Asn Ser Asn Thr Ile Ala His Thr Leu Ile Glu Lys Lys Lys Lys Asp
   305 310 315 320
   Gly Lys Asp Ile Gln Leu Thr Ile Asp Ala Lys Val Gln Lys Ser Ile
   325 330 335
   Tyr Asn Asn Met Lys Asn Asp Tyr Gly Ser Gly Thr Ala Ile His Pro
   340 345 350
   Gln Thr Gly Glu Leu Leu Ala Leu Val Ser Thr Pro Ser Tyr Asp Val
   355 360 365
   Tyr Pro Phe Met Tyr Gly Met Ser Asn Glu Glu Tyr Asn Lys Leu Thr
   370 375 380
   Glu Asp Lys Lys Glu Pro Leu Leu Asn Lys Phe Gln Ile Thr Thr Ser
   385 390 395 400
   Pro Gly Ser Thr Gln Lys Ile Leu Thr Ala Met Ile Gly Leu Asn Asn
   405 410 415
   Lys Thr Leu Asp Asp Lys Thr Ser Tyr Lys Ile Asp Gly Lys Gly Trp
   420 425 430
   Gln Lys Asp Lys Ser Trp Gly Gly Tyr Asn Val Thr Arg Tyr Glu Val
   435 440 445
   Val Asn Gly Asn Ile Asp Leu Lys Gln Ala Ile Glu Ser Ser Asp Asn
   450 455 460
   Ile Phe Phe Ala Arg Val Ala Leu Glu Leu Gly Ser Lys Lys Phe Glu
   465                 470                 475                 480
   Lys Gly Met Lys Lys Leu Gly Val Gly Glu Asp Ile Pro Ser Asp Tyr
                   485                 490                 495
   Pro Phe Tyr Asn Ala Gln Ile Ser Asn Lys Asn Leu Asp Asn Glu Ile
               500                 505                 510
   Leu Leu Ala Asp Ser Gly Tyr Gly Gln Gly Glu Ile Leu Ile Asn Pro
           515                 520                 525
   Val Gln Ile Leu Ser Ile Tyr Ser Ala Leu Glu Asn Asn Gly Asn Ile
       530                 535                 540
   Asn Ala Pro His Leu Leu Lys Asp Thr Lys Asn Lys Val Trp Lys Lys
   545                 550                 555                 560
   Asn Ile Ile Ser Lys Glu Asn Ile Asn Leu Leu Thr Asp Gly Met Gln
                   565                 570                 575
   Gln Val Val Asn Lys Thr His Lys Glu Asp Ile Tyr Arg Ser Tyr Ala
               580                 585                 590
   Asn Leu Ile Gly Lys Ser Gly Thr Ala Glu Leu Lys Met Lys Gln Gly
           595                 600                 605
   Glu Thr Gly Arg Gln Ile Gly Trp Phe Ile Ser Tyr Asp Lys Asp Asn
       610                 615                 620
   Pro Asn Met Met Met Ala Ile Asn Val Lys Asp Val Gln Asp Lys Gly
   625                 630                 635                 640
   Met Ala Ser Tyr Asn Ala Lys Ile Ser Gly Lys Val Tyr Asp Glu Leu
                   645                 650                 655
   Tyr Glu Asn Gly Asn Lys Lys Tyr Asp Ile Asp Glu
               660                 665